# EUROPEAN PATENT SPECIFICATION

(11) **EP 2 041 320 B1**
(45) Date of publication and mention of the grant of the patent: **16.03.2011**
(21) Application number: 07813073.9
(22) Date of filing: 18.07.2007
(51) Int. Cl.: C12Q 1/68

(54) **METHODS AND COMPOSITIONS FOR THE DIAGNOSIS AND TREATMENT OF CANCER**
VERFAHREN UND ZUSAMMENSETZUNGEN ZUR DIAGNOSE UND BEHANDLUNG VON KREBS
PROCÉDÉS ET COMPOSITIONS CONÇUS POUR LE DIAGNOSTIC ET LE TRAITEMENT DU CANCER

(30) Priority: 19.07.2006 US 807794 P
(43) Date of publication of application: 01.04.2009
(73) Proprietor: Genentech, Inc., South San Francisco CA 94080-4990 (US)
(72) Inventor: CHANT, John, Millbrae, California 94030 (US); GUERRERO, Anthony S., San Francisco, California 94110 (US); HAVERTY, Peter, San Francisco, California 94110 (US); HONCHELL, Cynthia, San Francisco, California 94122 (US); JUNG, Kenneth, San Francisco, California 94122 (US); WU, Thomas D., San Francisco, California 94110 (US)
(74) Representative: Walton, Seán Malcolm
(86) International application number: PCT/US2007/073811
(87) International publication number: WO 2008/011482

(56) References cited:
- WO-A-99/47152
- WO-A-03/020949
- SPITKOVSKII D D ET AL: "EXPRESSION OF CELL ONCOGENES IN HUMAN STOMACH TUMORS" VOPROSY ONKOLOGII (ST. PETERSBURG), vol. 31, no. 12, 1985, pages 44-49, XP009095374 ISSN: 0507-3758
- MELANI C ET AL: "INHIBITION OF PROLIFERATION BY C-MYB ANTISENSE OLIGODEOXYNUCLEOTIDES IN COLON ADENOCARCINOMA CELL LINES THAT EXPRESS C-MYB" CANCER RESEARCH, vol. 51, no. 11, 1991, pages 2897-2901, XP002467229 ISSN: 0008-5472
- QUEIMADO LURDES ET AL: "Identification of two distinct regions of deletion at 6q in gastric carcinoma" GENES CHROMOSOMES AND CANCER, vol. 14, no. 1, 1995, pages 28-34, XP002467230 ISSN: 1045-2257
- RAMSAY R G ET AL: "Myb expression is higher in malignant human colonic carcinoma and premalignant adenomatous polyps than in normal mucosa" CELL GROWTH AND DIFFERENTIATION, vol. 3, no. 10, 1992, pages 723-730, XP002467231 ISSN: 1044-9523
- MILNE ANYA N A ET AL: "Early-onset gastric cancers have a different molecular expression profile than conventional gastric cancers" MODERN PATHOLOGY, vol. 19, no. 4, April 2006 (2006-04), pages 564-572, XP002467232 ISSN: 0893-3952
- KODA T ET AL: "C-MYC GENE AMPLIFICATION IN PRIMARY STOMACH CANCER" JAPANESE JOURNAL OF CANCER RESEARCH, vol. 76, no. 7, 1985, pages 551-554, XP009095372 ISSN: 0910-5050

### Sequence Listing

<110> Genentech, Inc.
   Chant, John
   Guerrero, Anthony S.
   Haverty, Peter
   Honchell, Cynthia
   Jung, Kenneth
   Wu, Thomas
<120> METHODS AND COMPOSITIONS FOR THE DIAGNOSIS AND TREATMENT OF CANCER
<130> P2386R1 WO
<140> PCTUS0773811
   <141> 2007-07-18
<150> US 60/807,794
   <151> 2006-07-19
<160> 1
<210> 1
   <211> 640
   <212> PRT
   <213> Homo sapiens
<400> 1
   Met Ala Arg Arg Pro Arg His Ser Ile Tyr Ser Ser Asp Glu Asp
   1               5                   10                  15
   Asp Glu Asp Phe Glu Met Cys Asp His Asp Tyr Asp Gly Leu Leu
                   20                  25                  30
   Pro Lys Ser Gly Lys Arg His Leu Gly Lys Thr Arg Trp Thr Arg
                   35                  40                  45
   Glu Glu Asp Glu Lys Leu Lys Lys Leu Val Glu Gln Asn Gly Thr
                   50                  55                  60
   Asp Asp Trp Lys Val Ile Ala Asn Tyr Leu Pro Asn Arg Thr Asp
                   65                  70                  75
   Val Gln Cys Gln His Arg Trp Gln Lys Val Leu Asn Pro Glu Leu
                   80                  85                  90
   Ile Lys Gly Pro Trp Thr Lys Glu Glu Asp Gln Arg Val Ile Glu
                   95                  100                 105
   Leu Val Gln Lys Tyr Gly Pro Lys Arg Trp Ser Val Ile Ala Lys
                   110                 115                 120
   His Leu Lys Gly Arg Ile Gly Lys Gln Cys Arg Glu Arg Trp His
                   125                 130                 135
   Asn His Leu Asn Pro Glu Val Lys Lys Thr Ser Trp Thr Glu Glu
                   140                 145                 150
   Glu Asp Arg Ile Ile Tyr Gln Ala His Lys Arg Leu Gly Asn Arg
                   155                 160                 165
   Trp Ala Glu Ile Ala Lys Leu Leu Pro Gly Arg Thr Asp Asn Ala
                   170                 175                 180
   Ile Lys Asn His Trp Asn Ser Thr Met Arg Arg Lys Val Glu Gln
                   185                 190                 195
   Glu Gly Tyr Leu Gln Glu Ser Ser Lys Ala Ser Gln Pro Ala Val
                   200                 205                 210
   Ala Thr Ser Phe Gln Lys Asn Ser His Leu Met Gly Phe Ala Gln
                   215                 220                 225
   Ala Pro Pro Thr Ala Gln Leu Pro Ala Thr Gly Gln Pro Thr Val
                   230                 235                 240
   Asn Asn Asp Tyr Ser Tyr Tyr His Ile Ser Glu Ala Gln Asn Val
                   245                 250                 255
   Ser Ser His Val Pro Tyr Pro Val Ala Leu His Val Asn Ile Val
                   260                 265                 270
   Asn Val Pro Gln Pro Ala Ala Ala Ala Ile Gln Arg His Tyr Asn
                   275                 280                 285
   Asp Glu Asp Pro Glu Lys Glu Lys Arg Ile Lys Glu Leu Glu Leu
                   290                 295                 300
   Leu Leu Met Ser Thr Glu Asn Glu Leu Lys Gly Gln Gln Val Leu
                   305                 310                 315
   Pro Thr Gln Asn His Thr Cys Ser Tyr Pro Gly Trp His Ser Thr
                   320                 325                 330
   Thr Ile Ala Asp His Thr Arg Pro His Gly Asp Ser Ala Pro Val
                   335                 340                 345
   Ser Cys Leu Gly Glu His His Ser Thr Pro Ser Leu Pro Ala Asp
                   350                 355                 360
   Pro Gly Ser Leu Pro Glu Glu Ser Ala Ser Pro Ala Arg Cys Met
                   365                 370                 375
   Ile Val His Gln Gly Thr Ile Leu Asp Asn Val Lys Asn Leu Leu
                   380                 385                 390
   Glu Phe Ala Glu Thr Leu Gln Phe Ile Asp Ser Phe Leu Asn Thr
                   395                 400                 405
   Ser Ser Asn His Glu Asn Ser Asp Leu Glu Met Pro Ser Leu Thr
                   410                 415                 420
   Ser Thr Pro Leu Ile Gly His Lys Leu Thr Val Thr Thr Pro Phe
                   425                 430                 435
   His Arg Asp Gln Thr Val Lys Thr Gln Lys Glu Asn Thr Val Phe
                   440                 445                 450
   Arg Thr Pro Ala Ile Lys Arg Ser Ile Leu Glu Ser Ser Pro Arg
                   455                 460                 465
   Thr Pro Thr Pro Phe Lys His Ala Leu Ala Ala Gln Glu Ile Lys
                   470                 475                 480
   Tyr Gly Pro Leu Lys Met Leu Pro Gln Thr Pro Ser His Leu Val
                   485                 490                 495
   Glu Asp Leu Gln Asp Val Ile Lys Gln Glu Ser Asp Glu Ser Gly
                   500                 505                 510
   Ile Val Ala Glu Phe Gln Glu Asn Gly Pro Pro Leu Leu Lys Lys
                   515                 520                 525
   Ile Lys Gln Glu Val Glu Ser Pro Thr Asp Lys Ser Gly Asn Phe
                   530                 535                 540
   Phe Cys Ser His His Trp Glu Gly Asp Ser Leu Asn Thr Gln Leu
                   545                 550                 555
   Phe Thr Gln Thr Ser Pro Val Ala Asp Ala Pro Asn Ile Leu Thr
                   560                 565                 570
   Ser Ser Val Leu Met Ala Pro Ala Ser Glu Asp Glu Asp Asn Val
                   575                 580                 585
   Leu Lys Ala Phe Thr Val Pro Lys Asn Arg Ser Leu Ala Ser Pro
                   590                 595                 600
   Leu Gln Pro Cys Ser Ser Thr Trp Glu Pro Ala Ser Cys Gly Lys
                   605                 610                 615
   Met Glu Glu Gln Met Thr Ser Ser Ser Gln Ala Arg Lys Tyr Val
                   620                 625                 630
   Asn Ala Phe Ser Ala Arg Thr Leu Val Met
                   635                 640

## Description

### FIELD OF THE INVENTION

The present invention relates to methods and compositions for the diagnosis and treatment of cancers associated with gene amplification.

### BACKGROUND

Cancer is characterized by an increase in the number of abnormal, or neoplastic, cells derived from a normal tissue that proliferate and, under certain circumstances, invade adjacent tissues and eventually metastasize via the blood or lymphatic system. Alteration of gene expression is intimately related to uncontrolled cell growth and de-differentiation, which are common features of cancer. Certain cancers are characterized by overexpression of certain genes, *e.g*., oncogenes. A well known mechanism of gene overexpression in cancer cells is gene amplification. Gene amplification is a process in which multiple copies of one or more genes are produced in the chromosome of a cell. In certain instances, the process involves unscheduled replication of the region of the chromosome comprising those genes, followed by recombination of the replicated segments back into the chromosome (A litalo et al., Adv. Cancer Res., 47:235-281 [1986]). In certain cases, overexpression of a gene is correlated with gene amplification, *e.g*., is proportional to the number of copies made.

Amplification and/or overexpression of certain proto-oncogenes, e.g., those that encode growth factors and growth factor receptors, play important roles in the pathogenesis of various human malignancies. In certain instances, amplification and/or overexpression are associated with more malignant forms of cancer and thus may predict clinical outcome (Schwab et al., Genes Chromosomes Cancer, 1:181-193 [1990]; Alitalo *et al., supra*). For example, the human *erbB2* gene (also known as *her2* or *c-erbB-2),* which encodes a 185-kd transmembrane glycoprotein receptor (p185^{HER2} or HER2) related to the epidermal growth factor receptor EGFR, is overexpressed in about 25% to 30% of human breast cancers (Slamon et al., Science, 235:177-182 [1987]; Slamon et al., Science, 244:707-712 [1989]). Overexpression of *erbB2* is considered a predictor of a poor prognosis, especially in patients with primary disease that involves axillary lymph nodes (Slamon *et al.,* [1987] and [1989], *supra;* Ravdin and Chamness, Gene, 159:19-27 [1995]; and Hynes and Stern, Biochim. Biophys. Acta, 1198:165-184 [1994]). Overexpression of erbB2 has also been linked to sensitivity and/or resistance to certain hormone therapy and chemotherapeutic regimens, including CMF (cyclophosphamide, methotrexate, and fluoruracil) and anthracyclines (Baselga et al., Oncology, 11 (3 Suppl 1):43-48 [1997]). Patients that overexpress erbB2 show greater response to treatment with taxanes. *Id.*

Overexpression of *erb*B2 has provided the basis for targeted breast cancer therapies. A recombinant humanized anti-ErbB2 (anti-HER2) monoclonal antibody (Herceptin^{™}, Genentech, Inc.) has been successfully used to treat patients with ErbB2-overexpressing metastatic breast cancer. (Baselga et al., J. Clin. Oncol., 14:737-744 [1996]).

A continuing need exists for compositions and methods that target amplified genes and the products of those genes in the diagnosis and treatment of cancer.

A continuing need also exists for compositions and methods for the diagnosis and/or treatment of gastric (stomach) cancer. Approximately 90% to 95% of malignant gastric cancers are adenocarcinomas derived from epithelial cells of the stomach's innermost lining. Gastrointestinal stromal tumors (GISTs) are rare non-epithelial tumors derived from connective tissue. Approximately 70% of GISTs occur in the stomach. Over 800,000 people were diagnosed with gastric cancer, and over 630,000 people died of gastric cancer in the year 2000. The prognosis for gastric cancer is poor, with nearly half of patients having metastatic disease at the time of diagnosis. The 5-year survival rate is often less than 20%. *See* Ajani (2002) "gastric Cancer: Epidemiology and Therapy," in Encyclopedia of Cancer, vol. 2 (Elsevier Sciences, USA), pages 249-252.

Transcription of myb oncogene was detected in some gastric cancers but also in unrelated tissues and the authors concluded that the marker was unlikely to serve to diagnose such type of cancer (Spikoeskii et al Voprosy Onkologii vol. 31, No 12, 1985, pages 44-49).

The invention described herein meets the above-described needs and provides other benefits.

### SUMMARY

In one aspect, methods and compositions are provided for the diagnosis and treatment of gastric cancers associated with amplification and/or overexpression of the c-Myb gene.

In one aspect, a method of diagnosing the presence of a gastric cancer in a mammal is provided, the method comprising detecting whether the c-Myb gene is amplified in a test gastric sample from the mammal relative to a control sample, wherein amplification of the c-Myb gene indicates the presence of gastric cancer in the mammal. In one embodiment, detecting whether the c-Myb gene is amplified comprises detecting whether the copy number of the c-Myb gene is increased by at least 5-fold.

In another aspect, a method of diagnosing the presence of a gastric cancer in a mammal is provided, the method comprising detecting expression of the c-Myb gene in a test gastric sample from the mammal, wherein a higher level of c-Myb gene expression in the test gastric sample relative to a control sample indicates the presence of gastric cancer in the mammal. In one embodiment, detecting expression of the c-Myb gene comprises determining the level of mRNA transcription from the c-Myb gene. In one embodiment, a higher level of c-Myb gene expression comprises at least a 5-fold increase in mRNA transcription from the c-Myb gene in the test gastric sample relative to the control sample. In one embodiment, detecting expression of the c-Myb gene comprises determining the level of c-Myb. In one embodiment, detecting expression of the c-Myb gene comprises contacting the test gastric sample with an anti-c-Myb antibody and determining the level of expression of c-Myb in the test gastric sample by detecting binding of the anti-c-Myb antibody to c-Myb. In one embodiment, a higher level of c-Myb gene expression comprises at least a 5-fold increase in c-Myb levels.

In another aspect, a method of inhibiting the proliferation of a gastric cancer cell is provided, the method comprising exposing the cell to a c-Myb antagonist. In one embodiment, the c-Myb antagonist is an antisense nucleic acid of 10-30 nucleotides in length that binds to and reduces expression of a nucleic acid encoding c-Myb. In one embodiment, the antisense nucleic acid is an oligodeoxynucleotide. In one embodiment, the c-Myb antagonist is a nucleic acid that binds to the DNA binding domain of c-Myb. In one embodiment, the c-Myb antagonist is a small organic molecule that binds to c-Myb. In one embodiment, the c-Myb antagonist is a nucleic acid encoding an antibody that binds to c-Myb. In one embodiment, the antibody that binds to c-Myb is an antibody fragment. In one embodiment, the antibody that binds to c-Myb is an scFv.

In another aspect, a method of treating a gastric cancer associated with amplification or overexpression of the c-Myb gene is provided, the method comprising administering to an individual having the gastric cancer an effective amount of a pharmaceutical formulation comprising a c-Myb antagonist. In one embodiment, the c-Myb antagonist is an antisense nucleic acid of 10-30 nucleotides in length that binds to and reduces expression of a nucleic acid encoding c-Myb. In one embodiment, the antisense nucleic acid is an oligodeoxynucleotide. In one embodiment, the c-Myb antagonist is a nucleic acid that binds to the DNA binding domain of c-Myb. In one embodiment, the c-Myb antagonist is a small organic molecule that binds to c-Myb. In one embodiment, the c-Myb antagonist is a nucleic acid encoding an antibody that binds to c-Myb. In one embodiment, the antibody that binds to c-Myb is an antibody fragment. In one embodiment, the antibody that binds to c-Myb is an scFv.

In another aspect, a method for determining whether an individual having a gastric cancer will respond to a therapeutic that targets c-Myb or the c-Myb gene is provided, the method comprising determining whether the c-Myb gene is amplified in the colorectal cancer, wherein amplification of the c-Myb gene indicates that the individual will respond to the therapeutic. In one embodiment, the therapeutic is selected from an antisense nucleic acid; a nucleic acid that binds to the DNA binding domain of c-Myb; a small organic molecule that binds to c-Myb; and a nucleic acid encoding an antibody that binds to c-Myb.

### BRIEF DESCRIPTION OF THE FIGURES

Figure 1 shows the analysis of DNA copy number at a region of chromosome 6 comprising the c-Myb gene for nine gastric tumor samples (five gastrointestinal stromal tumors and four gastric adenocarcinomas).
Figure 2 shows analysis of DNA copy number and mRNA expression for two of the four adenocarcinomas depicted in Figure 1. Figure 2 also shows the locations of open reading frames that occur within the depicted region of chromosome 6.

### DETAILED DESCRIPTION OF EMBODIMENTS

Methods and compositions for the diagnosis and treatment of cancers associated with gene amplification are provided. In certain embodiments, the invention provides methods and compositions for the treatment of gastric cancer associated with amplification and/or overexpression of the c-Myb gene.

### I. DEFINITIONS

The phrases "gene amplification" and "gene duplication" (and variants such as "amplification of a gene" or "duplication of a gene") are used interchangeably and refer to a process by which multiple copies of a gene or gene fragment are formed in a particular cell or cell line. The duplicated region (a stretch of amplified DNA) is often referred to as an "amplicon." Usually, the amount of the messenger RNA (mRNA) produced, *i.e*., the level of gene expression, also increases in proportion to the number of copies made of the particular gene.

The term "c-Myb," as used herein, refers to any native c-Myb protein from any vertebrate source, including mammals such as primates (e.g. humans and monkeys) and rodents (e.g., mice and rats), unless otherwise indicated. The term encompasses "full-length," unprocessed c-Myb as well as any form of c-Myb that results from processing in the cell. The term also encompasses naturally occurring variants of c-Myb, e.g., splice variants, allelic variants, and other isoforms. The term also encompasses fragments or variants of a native c-Myb that maintain at least one biological activity of c-Myb. The term "c-Myb gene" refers to any gene from any vertebrate source that encodes a c-Myb protein, unless otherwise indicated.

The terms "cell proliferative disorder" and "proliferative disorder" refer to disorders that arc associated with some degree of abnormal cell proliferation. In one embodiment, the cell proliferative disorder is cancer.

"Tumor," as used herein, refers to all neoplastic cell growth and proliferation, whether malignant or benign, and all pre-cancerous and cancerous cells and tissues. The terms "cancer," "cancerous," "cell proliferative disorder," "proliferative disorder" and "tumor" are not mutually exclusive as referred to herein.

The terms "cancer" and "cancerous" refer to or describe the physiological condition in mammals that is typically characterized by unregulated cell growth/proliferation. Examples of cancer include, but arc not limited to, carcinoma, lymphoma (e.g., Hodgkin's and non-Hodgkin's lymphoma), blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioma, cervical cancer, ovarian cancer, liver cancer, bladder cancer, hepatoma, breast cancer, colon cancer, rectal cancer, gastric cancer, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer.

The term "gastric cancer" refers to stomach cancer and excludes esophageal cancer, cancer of the gastroesophageal junction, cancer of the small intestine, and colorectal cancer (any cancer of the large intestine and/or rectum), unless otherwise indicated.

The term "neoplasm" or "neoplastic cell" refers to an abnormal tissue or cell that proliferates more rapidly than corresponding normal tissues or cells and continues to grow after removal of the stimulus that initiated the growth.

A "gastric cancer cell" refers to a stomach cancer cell, either in vivo or in vitro, and encompasses cell lines derived from gastric cancer cells. In one embodiment, a gastric cancer cell is an adenocarcinoma.

As used herein, "treatment" (and variations such as "treat" or "treating") refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis.

An "individual" is a vertebrate. In certain embodiments, the vertebrate is a mammal. Mammals include, but are not limited to, farm animals (such as cows), sport animals, pets (such as cats, dogs, and horses), primates, mice and rats. In certain embodiments, a mammal is a human.

An "effective amount" refers to an amount effective, at dosages and for periods of time necessary, to achieve the desired therapeutic or prophylactic result.

A "therapeutically effective amount" of a substance/molecule of the invention may vary according to factors such as the disease state, age, sex, and weight of the individual, and the ability of the substance/molecule, to elicit a desired response in the individual. A therapeutically effective amount encompasses an amount in which any toxic or detrimental effects of the substance/molecule are outweighed by the therapeutically beneficial effects. A "prophylactically effective amount" refers to an amount effective, at dosages and for periods of time necessary, to achieve the desired prophylactic result. Typically, but not necessarily, since a prophylactic dose is used in subjects prior to or at an earlier stage of disease, the prophylactically effective amount would be less than the therapeutically effective amount.

The term "cytotoxic agent" as used herein refers to a substance that inhibits or prevents a cellular function and/or causes cell death or destruction. The term is intended to include radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu), chemotherapeutic agents (e.g., methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A "tumoricidal" agent causes destruction of tumor cells.

A "toxin" is any substance capable of having a detrimental effect on the growth or proliferation of a cell.

A "chemotherapeutic agent" is a chemical compound useful in the treatment of cancer. Examples of chemotherapeutic agents include alkylating agents such as thiotepa and CYTOXAN®) cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin: nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e. g., calicheamicin, especially calicheamicin gamma II and calicheamicin omegaII (see, e.g., Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., TAXOL® paclitaxel (Bristot-Myers Squibb Oncology, Princeton, N.J.), ABRAXANE^{™} Cremophor-free, albumin-engineered nanoparticle formulation of paclitaxel (American Pharmaceutical Partners, Schaumberg, Illinois), and TAXOTERE®) docetaxel (Rhône-Poulenc Rorer, Antony, France); chloranbucil; gemcitabine (GEMZAR®); 6-thioguanine; mercaptopurine; methotrexate; platinum analogs such as cisplatin and carboplatin; vinbtastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluoromethylornithine (DMFO); retinoids such as retinoic acid; capecitabine (XELODA®); pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN^{™}) combined with 5-FU and leucovovin.

Also included in this definition are anti-hormonal agents that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX®) tamoxifen), EVISTA® raloxifene, droloxifene, 4-hydroxytamoxifen, trioxifene, keoxifene, LY117018, onapristone, and FARESTON® toremifene; anti-progesterones; estrogen receptor down-regulators (ERDs); agents that function to suppress or shut down the ovaries, for example, leutinizing hormone-releasing hormone (LHRH) agonists such as LUPRON® and ELIGARD® leuprolide acetate, goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole. In addition, such definition of chemotherapeutic agents includes bisphosphonates such as clodronate (for example, BONEFOS® or OSTAC®), DIDROCAL® etidronate, NE-58095, ZOMETA® zoledronic acid/zoledronate, FOSAMAX® alendronate, AREDIA® pamidronate, SKELID® tiludronate, or ACTONEL® risedronate; as well as troxacitabine (a 1,3-dioxolane nucleoside cytosine analog); antisense oligonucleotides, particularly those that inhibit expression of genes in signaling pathways implicated in abherant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; LURTOTECAN® topoisomerase 1 inhibitor; ABARELIX® rmRH; lapatinib ditosylate (an ErbB-2 and EGFR dual tyrosine kinase small-molecule inhibitor also known as GW572016); and pharmaceutically acceptable salts, acids or derivatives of any of the above.

A "growth inhibitory agent" when used herein refers to a compound or composition which inhibits growth of a cell (such as a cell expressing c-Myb) either in vitro or in vivo. Thus, the growth inhibitory agent may be one which significantly reduces the percentage of cells (such as a cell expressing c-Myb) in S phase. Examples of growth inhibitory agents include agents that block cell cycle progression (at a place other than S phase), such as agents that induce G 1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

The term "antagonist" is used in the broadest sense, and includes any molecule that partially or fully blocks, inhibits, or neutralizes a biological activity of a polypeptide, such as c-Myb, or the transcription or translation thereof. Suitable antagonist molecules include, but are not limited to, antagonist antibodies, polypeptide fragments, oligopeptides, organic molecules (including small molecules), anti-sense nucleic acids, and nucleic acids encoding antagonist polypeptides.

"Antibodies" (Abs) and "immunoglobulins" (Igs) refer to glycoproteins having similar structural characteristics. While antibodies exhibit binding specificity to a specific antigen, immunoglobulins include both antibodies and other antibody-like molecules which generally lack antigen specificity. Polypeptides of the latter kind are, for example, produced at low levels by the lymph system and at increased levels by myelomas.

The terms "antibody" and "immunoglobulin" are used interchangeably in the broadest sense and include monoclonal antibodies (*e.g*., full length or intact monoclonal antibodies), polyclonal antibodies, monovalent antibodies, multivalent antibodies, multispecific antibodies (*e.g*., bispecific antibodies so long as they exhibit the desired biological activity) and may also include certain antibody fragments (as described in greater detail herein). An antibody can be chimeric, human, humanized and/or affinity matured.

The term "anti-c-Myb antibody" or "an antibody that binds to c-Myb" refers to an antibody that is capable of binding c-Myb with sufficient affinity such that the antibody is useful as a diagnostic and/or therapeutic agent in targeting c-Myb. Preferably, the extent of binding of an anti-c-Myb antibody to an unrelated, non-c-Myb protein is less than about 10% of the binding of the antibody to c-Myb as measured, e.g., by a radioimmunoassay (RIA). In certain embodiments, an antibody that binds to c-Myb has a dissociation constant (Kd) of ≤ 1µM, ≤ 100 nM, ≤ 10 nM, ≤ 1 nM, or ≤ 0.1 nM. In certain embodiments, an anti-c-Myb antibody binds to an epitope of c-Myb that is conserved among c-Myb from different species.

The terms "full length antibody," "intact antibody" and "whole antibody" are used herein interchangeably to refer to an antibody in its substantially intact form, not antibody fragments as defined below. The terms particularly refer to an antibody with heavy chains that contain the Fc region.

"Antibody fragments" comprise only a portion of an intact antibody, wherein the portion retains at least one, and as many as most or all, of the functions normally associated with that portion when present in an intact antibody. In one embodiment, an antibody fragment comprises an antigen binding site of the intact antibody and thus retains the ability to bind antigen. In another embodiment, an antibody fragment, for example, one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an *in vivo* half life substantially similar to an intact antibody. For example, such an antibody fragment may comprise an antigen binding arm linked to an Fc sequence capable of conferring *in vivo* stability to the fragment.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-combining sites and is still capable of cross-linking antigen.

"Fv" is a minimum antibody fragment which contains a complete antigen-binding site. In one embodiment, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three CDRs of each variable domain interact to define an antigen-binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment contains the heavy- and light-chain variable domains and also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains which enables the scFv to form the desired structure for antigen binding. For a review of scFv see Pluckthun, in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 404,097; W093/1161; Hudson et al. (2003) Nat. Med. 9:129-134; and Hollinger et al., Proc. Natl. Acad. Sci. USA 90: 6444-6448 (1993). Triabodies and tetrabodies are also described in Hudson et al. (2003) Nat. Med. 9:129-134.

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target binding sequence, to improve its production in cell culture, to reduce its immunogenicity *in vivo,* to create a multispecific antibody, etc., and that an antibody comprising the altered target binding sequence is also a monoclonal antibody of this invention. In contrast to polyclonal antibody preparations which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (e.g., Kohler et al., Nature, 256: 495 (1975); Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981)), recombinant DNA methods (see, e.g., U.S. Patent No. 4,816,567), phage display technologies (see, e.g., Clackson et al., Nature, 352: 624-628 (1991); Marks et al., J. Mol. Biol. 222: 581-597 (1992); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101 (34): 12467-12472 (2004); and Lee et al., J. Immunol. Methods 284(1-2): 119-132(2004), and technologies for producing human or human-like antibodies in animals that have parts or all of the human immunoglobulin loci or genes encoding human immunoglobulin sequences (see, e.g., WO98/24893; WO96/34096; WO96/33735; WO91/10741; Jakobovits et al., Proc. Natl. Acad. Sci. USA 90: 2551 (1993); Jakobovits et al., Nature 362: 255-258 (1993); Bruggemann et al., Year in Immunol. 7:33 (1993); U.S. Patent Nos. 5,545,807; 5,545,806; 5,569,825; 5,625,126; 5,633,425; 5,661,016; Marks et al., Bio. Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996) and Lonberg and Huszar, Intern. Rev. Immunol. 13: 65-93 (1995).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (e.g., murine) antibodies are chimeric antibodies that contain minimal sequence derived from non-human immunoglobulin. In one embodiment, a humanized antibody is a human immunoglobulin (recipient antibody) in which residues from a hypervariable region of the recipient are replaced by residues from a hypervariable region of a non-human species (donor antibody) such as mouse, rat, rabbit, or nonhuman primate having the desired specificity, affinity, and/or capacity. In some instances, framework region (FR) residues of the human immunoglobulin are replaced by corresponding non-human residues. Furthermore, humanized antibodies may comprise residues that are not found in the recipient antibody or in the donor antibody. These modifications may be made to further refine antibody performance. In general, a humanized antibody will comprise substantially all of at least one, and typically two, variable domains, in which all or substantially all of the hypervariable loops correspond to those of a non-human immunoglobulin, and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994).

A "human antibody" is one which comprises an amino acid sequence corresponding to that of an antibody produced by a human and/or has been made using any of the techniques for making human antibodies as disclosed herein. Such techniques include screening human-derived combinatorial libraries, such as phage display libraries (see, e.g., Marks et al., J. Mol. Biol., 222: 581-597 (1991) and Hoogenboom et al., Nucl. Acids Res., 19: 4133-4137 (1991)); using human myeloma and mouse-human heteromyeloma cell lines for the production of human monoclonal antibodies (see, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991)); and generating monoclonal antibodies in transgenic animals (e.g., mice) that are capable of producing a full repertoire of human antibodies in the absence of endogenous immunoglobulin production (see, e.g., Jakobovits et al., Proc. Natl. Acad. Sci USA, 90: 2551 (1993); Jakobovits et al., Nature, 362: 255 (1993); Bruggermann et al., Year in Immunol., 7: 33 (1993)). This definition of a human antibody specifically excludes a humanized antibody comprising antigen-binding residues from a non-human animal.

An "affinity matured" antibody is one with one or more alterations in one or more CDRs thereof which result in an improvement in the affinity of the antibody for antigen, compared to a parent antibody which does not possess those alteration(s). In one embodiment, an affinity matured antibody has nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of HVR and/or framework residues is described by: Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et. al, J. Mol. Biol. 226:889-896 (1992).

A "blocking" antibody or an "antagonist" antibody is one which inhibits or reduces a biological activity of the antigen it binds. Certain blocking antibodies or antagonist antibodies partially or completely inhibit the biological activity of the antigen.

Antibody "effector functions" refer to those biological activities attributable to the Fc region (a native sequence Fc region or amino acid sequence variant Fc region) of an antibody, and vary with the antibody isotype. Examples of antibody effector functions include: Clq binding and complement dependent cytotoxicity; Fc receptor binding; antibody-dependent cell-mediated cytotoxicity (ADCC); phagocytosis; down regulation of cell surface receptors (*e.g.* B cell receptor); and B cell activation.

"Fc receptor" or "FcR" describes a receptor that binds to the Fc region of an antibody. In some embodiments, an FcR is a native human FcR. In some embodiments, an FcR is one which binds an IgG antibody (a gamma receptor) and includes receptors of the FcγRI, FcγRII, and FcγRIII subclasses, including allelic variants and alternatively spliced forms of those receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain (see Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

The term "Fc receptor" or "FcR" also includes the neonatal receptor, FcRn, which is responsible for the transfer of maternal IgGs to the fetus (Guyer et al., J. Immunol. 117:587 (1976) and Kim et al., J. Immunol. 24:249 (1994)) and regulation of homeostasis of immunoglobulins. Methods of measuring binding to FcRn are known. Binding to human FcRn in vivo and serum half life of human FcRn high affinity binding polypeptides can be assayed, e.g., in transgenic mice or transfected human cell lines expressing human FcRn, or in primates administered with Fc variant polypeptides.

WO00/42072 (Presta) describes antibody variants with improved or diminished binding to FcRs. See, also, Shields et al. J. Biol. Chem. 9(2): 6591-6604 (2001).

"Human effector cells" are leukocytes which express one or more FcRs and perform effector functions. In certain embodiments, the cells express at least FcγRIII and perform ADCC effector function(s). Examples of human leukocytes which mediate ADCC include peripheral blood mononuclear cells (PBMC), natural killer (NK) cells, monocytes, cytotoxic T cells and neutrophils. The effector cells may be isolated from a native source, e.g., from blood.

"Antibody-dependent cell-mediated cytotoxicity" or "ADCC" refers to a form of cytotoxicity in which immunoglobulin bound to Fc receptors (FcRs) present on certain cytotoxic effector cells (*e.g*. Natural Killer (NK) cells, neutrophils, and macrophages) enables those cytotoxic effector cells to bind specifically to an antigen-bearing target cell and subsequently kill the target cell with cytotoxins. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 or Presta U.S. Patent No. 6,737,056 may be performed. Useful effector cells for such assays include peripheral blood mononuclear cells (PBMC) and Natural Killer (NK) cells. Alternatively, or additionally, ADCC activity of the molecule of interest may be assessed *in vivo,* e.g., in an animal model such as that disclosed in Clynes et al. PNAS (USA) 95:652-656 (1998).

"Complement dependent cytotoxicity" or "CDC" refers to the lysis of a target cell in the presence of complement. Activation of the classical complement pathway is initiated by the binding of the first component of the complement system (Clq) to antibodies (of the appropriate subclass) which are bound to their cognate antigen. To assess complement activation, a CDC assay, *e.g*. as described in Gazzano-Santoro et al., J. Immunol. Methods 202:163 (1996), may be performed.

Polypeptide variants with altered Fc region amino acid sequences and increased or decreased Clq binding capability are described in US Patent No. 6,194,551B1 and WO99/51642. See, also, Idusogie et al. J. Immunol. 164: 4178-4184 (2000).

The term "Fc region-comprising polypeptide" refers to a polypeptide, such as an antibody or immunoadhesin, which comprises an Fc region. The C-terminal lysine (residue 447 according to the EU numbering system) of the Fc region may be removed, for example, during purification of the polypeptide or by recombinant engineering the nucleic acid encoding the polypeptide. Accordingly, a composition comprising a polypeptide having an Fc region according to this invention can comprise polypeptides with K447, with all K447 removed, or a mixture of polypeptides with and without the K447 residue.

A "cytotoxic antibody" is an antibody that is capable of an effector function and/or inducing cell death upon binding to its target antigen.

An "immunoconjugate" refers to an antibody conjugated to one or more cytotoxic agents.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (*i.e*., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

A "small molecule" or "small organic molecule" is defined herein as an organic molecule having a molecular weight below about 500 Daltons.

A "c-Myb-binding oligopeptide" or an "oligopeptide that binds c-Myb" is an oligopeptide that is capable of binding c-Myb with sufficient affinity such that the oligopeptide is useful as a diagnostic and/or therapeutic agent in targeting c-Myb. In certain embodiments, the extent of binding of a c-Myb-binding oligopeptide to an unrelated, non-c-Myb protein is less than about 10% of the binding of the c-Myb-binding oligopeptide to c-Myb as measured, e.g., by a surface plasmon resonance assay. In certain embodiments, a c-Myb-binding oligopeptide has a dissociation constant (Kd) of ≤ 1µM, ≤ 100 nM, ≤ 10 nM, ≤ 1 nM, or ≤ 0.1 nM.

A "c-Myb-binding organic molecule" or "an organic molecule that binds c-Myb" is an organic molecule other than an oligopeptide or antibody as defined herein that is capable of binding c-Myb with sufficient affinity such that the organic molecule is useful as a diagnostic and/or therapeutic agent in targeting c-Myb. In certain embodiments, the extent of binding of a c-Myb-binding organic molecule to an unrelated, non-c-Myb protein is less than about 10% of the binding of the c-Myb-binding organic molecule to c-Myb as measured, e.g., by a surface plasmon resonance assay. In certain embodiments, a c-Myb-binding organic molecule has a dissociation constant (Kd) of ≤ 1µM, ≤ 100 nM, ≤ 10 nM, ≤ 1 nM, or ≤ 0.1 nM.

The dissociation constant (Kd) of any molecule that binds a target polypeptide may conveniently be measured using a surface plasmon resonance assay. Such assays may employ a BIAcore^{™}-2000 or a BIAcore^{™}-3000 (BIAcore, Inc., Piscataway, NJ) at 25°C with immobilized target polypeptide CM5 chips at ∼10 response units (RU). Briefly, for example, carboxymethylated dextran biosensor chips (CM5, BIAcore Inc.) are activated with *N*-ethyl-*N*'- (3-dimethylaminopropyl)-carbodiimide hydrochloride (EDC) and N-hydroxysuccinimide (NHS) according to the supplier's instructions. Target polypeptide is diluted with 10 mM sodium acetate, pH 4.8, to 5 µg/ml (∼0.2 µM) before injection at a flow rate of 5 µl/minute to achieve approximately 10 response units (RU) of coupled protein. Following the injection of target polipeptide, 1 M ethanolamine is injected to block unreacted groups. For kinetics measurements, two-fold serial dilutions of the binding molecule (0.78 nM to 500 nM) are injected in PBS with 0.05% Tween 20 (PBST) at 25°C at a flow rate of approximately 25 µl/min. Association rates (kₒₙ) and dissociation rates (k_{off}) are calculated using a simple one-to-one Langmuir binding model (BIAcore Evaluation Software version 3.2) by simultaneously flitting the association and dissociation sensorgrams. The equilibrium dissociation constant (Kd) is calculated as the ratio k_{off}/kₒₙ. See, e.g., Chen, Y., et al., (1999) J. Mol. Biol. 293:865-881. If the on-rate of an antibody exceeds 10⁶ M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM antibody (Fab form) in PBS, pH 7.2, in the presence of increasing concentrations of antigen as measured in a spectrometer, such as a stop-flow equipped spectrophometer (Aviv Instruments) or a 8000-series SLM-Aminco spectrophotometer (ThermoSpectronic) with a stirred cuvette.

A "liposome" is a small vesicle composed of various types of lipids, phospholipids and/or surfactant which is useful for delivery of an agent, e.g., a drug, to a mammal. The components of the liposome are commonly arranged in a bilayer formation, similar to the lipid arrangement of biological membranes.

The word "label" when used herein refers to a detectable compound or composition. The label may be detectable by itself (*e.g*., radioisotope labels or fluorescent labels) or, in the case of an enzymatic label, may catalyze chemical alteration of a substrate compound or composition which results in a detectable product. Radionuclides that can serve as detectable labels include, for example, I-131, I-123, I-125, Y-90, Re-188, Re-186, At-211, Cu-67, Bi-212, and Pd-109.

An "isolated" biological molecule, such as a nucleic acid, polypeptide, or antibody, is one which has been identified and separated and/or recovered from at least one component of its natural environment.

### II. EMBODIMENTS OF THE INVENTION

Methods and compositions for the diagnosis and treatment of cancers associated with gene amplification and/or overexpression are provided. In one aspect, methods and compositions for the diagnosis and treatment of gastric cancer are provided. Those methods and compositions are based, in part, on the discovery that a region of chromosome 6 comprising the c-Myb gene is amplified in certain gastric cancers, and this amplification is correlated with increased expression of c-Myb mRNA.

c-Myb was first identified as a cellular counterpart of a viral oncogene. Accordingly, c-Myb is a "protooncogene" that may be converted into an oncogenic form, e.g., by overexpression or by mutation, such as N- and/or C-terminal truncation. *See* Ramsay et al. (2003) Expert Opin. Ther. Targets 7(2):235-248; Gewirtz (1999) Oncogene 18:3056-3062.

c-Myb is a transcription factor having distinct, conserved functional domains. It contains an N-terminal DNA binding domain having three imperfect tandem repeats that maintain a helix-turn-helix structure. It also contains a central acidic transactivation domain, and a C-terminal negative regulatory domain. *See* Majello et al. (1986) Proc. Natl. Acad. Sci. U.S.A. 83:9636-9640; Ness (1999) Oncogene 18:3039-3046; Ramsay et al. (2003) Expert Opin. Ther. Targets 7(2):235-248. A full length form of human c-Myb is shown in SEQ ID NO:1. That sequence contains the following features:

**TABLE 1**

| **Feature** | **Amino Acid Residues** |
|---|---|
| DNA binding domain repeats | 35-86 |
| | 87-138 |
| | 139-189 |
| Transactivation domain | 275-327 |
| Negative regulatory domain | 328-465 |
| Leucine zipper | 367-397 |

Alternative splicing of c-Myb mRNA generates various isoforms, including isoforms having insertions, deletions, or substitutions in the central and/or C-terminal portions of the protein. *See, e.g.,* Westin et al. (1990) "Alternative splicing of the huma c-Myb gene," µ 5:1117-1124; Dasgupta et al. (1989) "Identification of alternatively spliced transcripts for huma c-Myb: molecular cloning and sequence analysis of huma c-Myb exon 9A sequences," Oncogene 4:1419-1423; and NCBI Accession No. U22376.1. Additionally, the chromosomal location of the c-Myb gene is 6q22-23 on the short arm of chromosome 6.

### A. Methods of Diagnosis and Detection

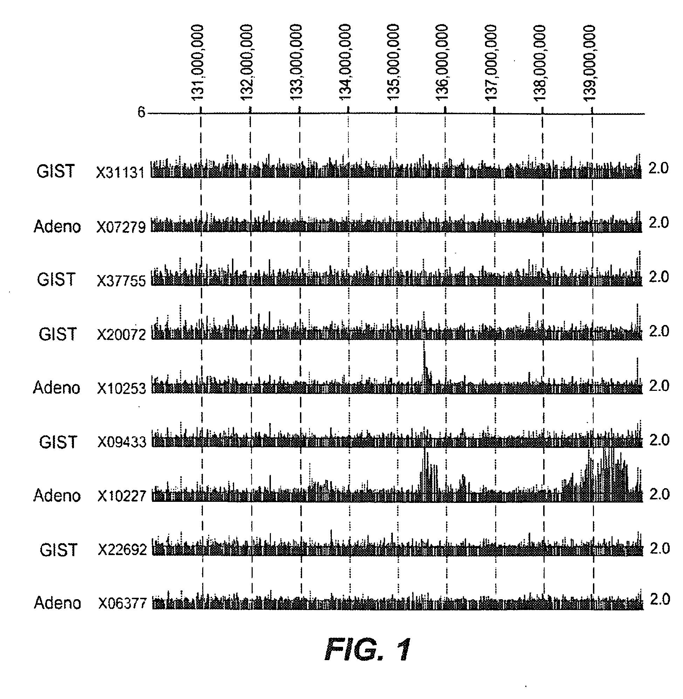

In one aspect, methods of diagnosing gastric cancer are provided. As described below in the Examples, gastric tumors were discovered in which a region of chromosome 6 was amplified. The c-Myb gene falls squarely within the region of amplification, as shown in Figure 1, and is thus an attractive target for gastric cancer diagnostics and therapeutics.

Accordingly, in one aspect, a methods of diagnosing the presence of a gastric cancer in a mammal is provided, the method comprising detecting whether the c-Myb gene is amplified in a test gastric sample from the mammal relative to a control sample, wherein amplification of the c-Myb gene indicates the presence of gastric cancer in the mammal. As used herein, the term "detecting" encompasses quantitative or qualitative detection. A "test gastric sample" is a biological sample derived from gastric tissue that may or may not be cancerous, e.g., a sample of gastric cells suspected of being cancerous or a whole cell extract or fractionated extract (such as a nuclear extract) derived from gastric cells. A "control sample" is a biological sample derived from (a) normal tissue, e.g., normal gastric cells or a whole cell extract or fractionated extract (such as a nuclear extract) derived from such cells, or (b) gastric cancer tissue in which the c-Myb gene is known not to be amplified or overexpressed, or a whole cell extract or fractionated extract derived therefrom. The c-Myb gene is said to be "amplified" if the copy number of the c-Myb gene is increased by at least 3-, 4-, 5-, 7-, 10-, 15-, 20-, 25-, 30-, 35-, 40-, 45-, or 50-fold in the test gastric sample relative to the control sample.

In certain embodiments, detecting amplification of the c-Myb gene is achieved using certain techniques known to those skilled in the art. For example, comparative genome hybridization may be used to produce a map of DNA sequence copy number was a function of chromosomal location. See, e.g., Kallionicmi et al. (1992) Science 258:818-821. Amplification of the c-Myb gene may also be detected, e.g., by Southern hybridization using a probe specific for the c-Myb gene or by real-time quantitative PCR.

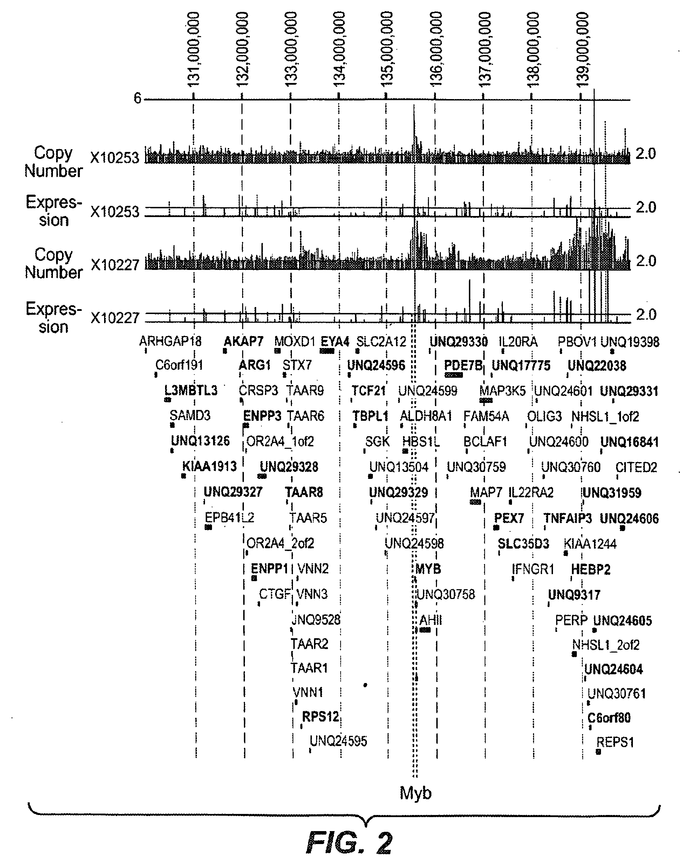

In certain embodiments, detecting amplification of the c-Myb gene is achieved by directly assessing the copy number of the c-Myb gene, for example, by using a probe that hybridizes to the c-Myb gene. In certain embodiments, detecting amplification of the c-Myb gene is achieved by indirectly assessing the copy number of the c-Myb gene, for example, by assessing the copy number of a chromosomal region that lies outside the c-Myb gene but is co-amplified with the c-Myb gene. Guidance for selecting such a region is provided, e.g., in Figures 1 and 2.

In another aspect, a method of diagnosing the presence of a gastric cancer in a mammal is provided, the method comprising detecting expression of the c-Myb gene in a test gastric sample from the mammal, wherein a higher level of c-Myb gene expression in the test gastric sample relative to a control sample indicates the presence of gastric cancer in the mammal. In certain embodiments, expression of the c-Myb gene is detected by determining the level of mRNA transcription from the c-Myb gene. Levels of mRNA transcription may be determined, either quantitatively or qualitatively, by various methods known to those skilled in the art. Levels of mRNA transcription may also be determined directly or indirectly by detecting levels of cDNA generated from the mRNA. Exemplary methods for determining levels of mRNA transcription include, but are not limited to, real-time quantitative RT-PCR and hybridization-based assays, including microarray-based assays and fitter-based assays such as Northern blots. In certain embodiments, "a higher level of c-Myb gene expression" means at least a 3-, 4-, 5-, 7-, 10-, 15-, 20-, 25-, 30-, 35-, 40-, 45-, or 50-fold increase in mRNA transcription from the c-Myb gene.

In other embodiments, expression of the c-Myb gene is detected by determining the level of c-Myb. Levels of c-Myb may be determined, either quantitatively or quantitatively, by certain methods known to those skilled in the art, including antibody-based detection methods. In one embodiment, detecting expression of the c-Myb gene in a test gastric sample comprises contacting the test gastric sample with an anti-c-Myb antibody and determining the level of expression (either quantitatively or qualitatively) of c-Myb in the test gastric sample by detecting binding of the anti-c-Myb antibody to c-Myb. In certain embodiments, binding of an anti-c-Myb antibody to c-Myb may be detected by various methods known to those skilled in the art including, but not limited to, fluorescence activated cell sorting, Western blot, radioimmunoassay, ELISA, and the like. In certain embodiments, "a higher level of c-Myb gene expression" means at least a 3-, 4-, 5-, 7-, 10-, 15-, 20-, 25-, 30-, 35-, 40-, 45-, or 50-fold increase in c-Myb levels.

For any of the above methods, the stated purpose of "diagnosing the presence of a gastric cancer in a mammal" is nonlimiting and encompasses classifying the type of gastric cancer present in a mammal by detecting whether the c-Myb gene is amplified and/or expressed at a higher level in a test sample of gastric cancer relative to a control sample. Classifying a gastric cancer based on whether or not the c-Myb gene is amplified and/or overexpressed is useful, e.g., for determining whether an individual have a gastric cancer will respond to a therapeutic agent that targets c-Myb or the c-Myb gene, and thus, for selecting the optimal regimen for treating the gastric cancer, as further described below.

For example, a method is provided herein for determining whether an individual having a gastric cancer will respond to a therapeutic that targets c-Myb or the c-Myb gene, the method comprising determining whether the c-Myb gene is amplified and/or overexpressed in the gastric cancer (e.g., by using any of the methods described above), wherein amplification and/or overexpression of the c-Myb gene indicates that the individual will respond to the therapeutic. A "therapeutic that targets c-Myb or the c-Myb gene" means any agent that affects the expression and/or an activity of c-Myb or the c-Myb gene including, but not limited to, any of the c-Myb antagonists described below, Part B, including such therapeutics that are already known in the art as well as those that are later developed.

### B. Compositions and Pharmaceutical Formulations

Pharmaceutical formulations for treating gastric cancer are provided. In certain embodiments, a pharmaceutical formulation comprises at least one c-Myb antagonist, a pharmaceutically acceptable carrier, and optionally, at least one additional therapeutic agent. A c-Myb antagonist may be any molecule that partially or fully blocks, inhibits, or neutralizes 1) a biological activity of c-Myb, e.g., the transcriptional activation activity or DNA binding activity of c-Myb, or 2) the transcription or translation of c-Myb. In certain embodiments, a c-Myb antagonist comprises an anti-c-Myb antibody or a nucleic acid encoding an anti-c-Myb antibody; an oligopeptide; an organic molecule; a nucleic acid that binds to c-Myb; or an antisense nucleic acid, such as an antisense oligodeoxynucleotide.

In other embodiments, a pharmaceutical formulation comprises at least one cytotoxic anti-c-Myb antibody, pharmaceutically acceptable carrier, and optionally, at least one additional therapeutic agent. In yet other embodiments, a pharmaceutical formulation comprises at least one immunoconjugate, wherein the immunoconjugate comprises an antibody that binds c-Myb and a cytotoxic agent; a pharmaceutically acceptable carrier; and optionally, at least one additional therapeutic agent.

### 1. c-Myb antagonists

In one aspect, a c-Myb antagonist is an anti-c-Myb antibody. In certain embodiments, an anti-c-Myb antibody is a "blocking antibody," e.g, an antibody that fully or partially blocks an activity of c-Myb. In certain embodiments, an anti-c-Myb antibody binds to the DNA binding domain of a c-Myb. An exemplary DNA binding domain of a human c-Myb is shown in Table 1. In certain embodiments, an anti-c-Myb antibody binds to the transactivation domain of a c-Myb. An exemplary transactivation domain of human c-Myb is shown in Table 1.

In one aspect, a c-Myb antagonist is a nucleic acid encoding an anti-c-Myb antibody. Certain blocking anti-c-Myb antibodies are known in the art and have been expressed in cells by transfecting the cells with nucleic acid encoding the antibodies. The antibodies thus expressed were effective in reducing c-Myb activity in the cell. *See, e.g.,* Afroze et al. (2003) Am. J. Physiol. Cell Physiol. 285:C88-C95; Kasono et al. (1998) Biochem Biophys Res Commun. 251(1):124-30*.* In particular, Kasono et al. described expression of an anti-c-Myb scFv that functionally knocked out c-Myb and had a cytotoxic effect on a Myb-expressing leukemia cell line, thus suggesting a utility for this antibody in gene therapy. *Id.*

In various embodiments of the invention, an anti-c-Myb antibody (including antagonist anti-c-Myb antibodies and cytotoxic anti-c-Myb antibodies, discussed below, Part 2) is a monoclonal antibody. In various embodiments, an anti-c-Myb antibody is an antibody fragment, e.g., a Fab, Fab'-SH, Fv, scFv, or (Fab')₂ fragment, or a single domain antibody (Domantis, Inc., Waltham, MA; *see, e.g.,* US Pat. No. 6,248,516 B1). In certain embodiments, an anti-c-Myb antibody is a bispecific antibody (see, e.g., WO94/04690 and Suresh et al. (1986) Methods in Enzymology 121:210). In certain embodiments, an anti-c-Myb antibody is a chimeric, humanized, or human antibody.

In another aspect, a c-Myb antagonist is an oligopeptide that binds to a c-Myb. In one embodiment, an oligopeptide binds to the DNA binding domain of a c-Myb. In one embodiment, an oligopeptide binds to the transactivation domain of a c-Myb. Oligopeptides may be chemically synthesized using known oligopeptide synthesis methodology or may be prepared and purified using recombinant technology. Such oligopeptides are usually at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length. Such oligopeptides may be identified without undue experimentation using well known techniques. In this regard, it is noted that techniques for screening oligopeptide libraries for oligopeptide that are capable of specifically binding to a polypeptide target are well known in the art (see, e.g., U.S. Patent Nos. 5,556,762, 5,750,373, 4,708,871, 4,833,092, 5,223,409, 5,403,484, 5,571,689, 5,663,143; PCT Publication Nos. WO 84/03506 and WO84/03564; Geysen et al., Proc. Natl. Acad. Sci. U.S.A., 81:3998-4002 (1984); Geysen et al., Proc. Natl. Acad. Sci. USA, 82:178-182 (1985); Geysen et al., in Synthetic Peptides as Antigens, 130-149 (1986); Geysen et al., J. Immunol. Meth., 102:259-274 (1987); Schoofs et al., J. Immunol., 140:611-616 (1988), Cwirla, S. E. et al. (1990) Proc. Natl. Acad. Sci. USA, 87:6378; Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363, and Smith, G. P. (1991) Current Opin. Biotechnol., 2:668). In certain embodiments, an oligopeptide may be conjugated to a cytotoxic agent.

In yet another aspect, a c-Myb antagonist is an organic molecule that binds to c-Myb, other than an oligopeptide or antibody as described herein. An organic molecule may be, for example, a small molecule. In one embodiment, an organic molecule binds to the DNA binding domain of c-Myb. In another embodiment, an organic molecule binds to the transactivation domain of c-Myb. In another embodiment, an organic molecule blocks transcription of c-Myb. Certain of such molecules are known in the art. For example, it is known that the c-Myb gene is transcribed by RNA polymerase II, which pauses at a poly-T tract of 19-20 residues in the first intron. *See* Ramsay et al. (2003) Expert Opin. Ther. Targets 7(2):235-248*.* This pausing may be enhanced by exposing cells to agents that promote differentiation, such as DMSO, or that inhibit histone deacetylation, such as sodium butyrate hexamethylene bisacetamide (HMBA), and suberoylanilide hydroxamic acid (SAHA). *Id*.

An organic molecule that binds to c-Myb may be identified and chemically synthesize using known methodology (see, e.g., PCT Publication Nos. WO00/00823 and WO00/39585). Such organic molecules are usually less than about 2000 daltons in size, alternatively less than about 1500, 750, 500, 250 or 200 daltons in size, wherein such organic molecules that arc capable of binding to c-Myb may be identified without undue experimentation using well known techniques. In this regard, it is noted that techniques for screening organic molecule libraries for molecules that are capable of binding to a polypeptide target arc well known in the art (see, e.g., PCT Publication Nos. WO00/00823 and WO00. 39585). In certain embodiments, an organic molecule may be conjugated to a cytotoxic agent.

In yet another aspect, a c-Myb antagonist is a nucleic acid. In certain embodiments, a nucleic acid encodes a polypeptide antagonist of c-Myb, e.g., an antibody that binds c-Myb. In certain embodiments, a nucleic acid comprises a sequence that binds to the DNA binding domain of c-Myb. In one of such embodiments, the nucleic acid comprises a sequence derived from a natural c-Myb binding site, e.g., all or a portion of a natural c-Myb binding site or a variant thereof that retains the ability to bind c-Myb. Such a nucleic acid may be used, e.g., to titrate out c-Myb from its natural binding site. The nucleic acid sequences of various c-Myb binding sites have been identified (*see* Lang et al. (2005) Oncogene 24:1375-1384). In certain exemplary embodiments, a c-Myb antagonist may be a nucleic acid comprising any of those sequences or a fragment of variant thereof that retains the ability to bind c-Myb.

In yet another aspect, a c-Myb antagonist is an antisense nucleic acid that decreases expression of the c-Myb gene (i.e., that decreases transcription of the c-Myb gene and/or translation of c-Myb mRNA. In certain embodiments, an antisense nucleic acid binds to a nucleic acid (DNA or RNA) encoding c-Myb. In certain embodiments, an antisense nucleic acid is an oligonucleotide of about 10-30 nucleotides in length (including all points between those endpoints). In certain embodiments, an antisense nucleic acid is an oligonucleotide of about 18-24 nucleotides in length (including all points between those endpoints). In certain embodiments, an antisense oligonucleotide comprises a modified sugar-phosphodiester backbones (or other sugar linkages, including phosphorothioate linkages and linkages as described in WO 91/06629), wherein such modified sugar-phosphodiester backbones are resistant to endogenous nucleases.

In one embodiment, an antisense nucleic acid is an oligodeoxyribonucleotide (ODN), which results in the degradation and/or reduced transcription or translation of c-Myb mRNA. In certain embodiments, phosphothioate-linked ODNs (PS-ODNs) are preferred. Certain examples of c-Myb-specific ODNs are known to those skilled in the art and are described, e.g., in Ramsay et al. (2003) Expert Opin. The. Targets 7(2):235-248. Certain ODNs target the translation start site of c-Myb RNA and regions downstream or, preferably, upstream of the translation start site. *Id*. ODNs targeting c-Myb have been successfully delivered in a cell-type specific manner *in vivo* and *in vitro* using cationic liposome preparations. *Id***.** ; Brignole et al. (2003) Cancer Lett. 197:231-235. Indeed, ODNs targeting c-Myb have been administered in clinical trials for treating hematopoietic cancer using *ex vivo* methods. *See* Gewirtz (1999) Oncogene 18:3056-3062.

In certain embodiments, an antisense nucleic acid is an RNA that reduces expression of a target nucleic acid by "RNA interference" ("RNAi"). For review of RNAi, see, e.g., Novina et al. (2004) Nature 430:161-164. Such RNAs are derived from, for example, short interfering RNAs (siRNAs) and microRNAs. siRNAs, e.g., may be synthesized as double stranded oligoribonucleotides of about 18-26 nucleotides in length. *Id*. Thus, antisense nucleic acids that decrease expression of c-Myb are well within the skill in the art.

In yet another aspect, a c-Myb antagonist is a triple-helix forming ODN. In yet another aspect, a c-Myb antagonist is a ribozyme that targets c-Myb mRNA. Triple-helix forming ODNs and ribozymes are described, e.g., in Gewirtz (1999) Oncogene 18:3056-3062; Gunther et al. (1996) Photochem. Photobiol. 63:207-212; James et al. (1997) Methods Mol. Biol. 74:1-9.

### 2. Cytotoxic Antibodies

In one aspect, cytotoxic antibodies are provided. In certain embodiments, a cytotoxic antibody is an anti-c-Myb antibody, such as those provided above, which effects an effector function and/or induces cell death.

### 3. Immunoconjugates

Immunoconjugates, or "antibody-drug conjugates," are useful for the local delivery of cytotoxic agents in the treatment of cancer. *See, e.g.,* Syrigos et al. (1999) Anticancer Research 19:605-614; Niculescu-Duvaz et al. (1997) Adv. Drug Deliv. Rev. 26:151-172; U.S. Pat. No. 4,975,278. Immunoconjugates allow for the targeted delivery of a drug moiety to a tumor, whereas systemic administration of unconjugated cytotoxic agents may result in unacceptable levels of toxicity to normal cells as well as the tumor cells sought to be eliminated. *See.* Baldwin et al. (Mar. 15, 1986) Lancet pp. 603-05; Thorpe (1985) "Antibody Carriers Of Cytotoxic Agents In Cancer Therapy: A Review," in Monoclonal Antibodies '84: Biological and Clinical Applications (A. Pinchera et al., eds.) pp. 475-506.

In one aspect, an immunoconjugate comprises an antibody that binds c-Myb, such as those provided above, and a cytotoxic agent, such as a chemotherapeutic agent, a growth inhibitory agent, a toxin (*e.g*., an enzymatically active toxin of bacterial, fungal, plant, or animal origin, or fragments thereof), or a radioactive isotope (*i.e.,* a radioconjugate). In one aspect, an antagonist of c-Myb is a nucleic acid that encodes an immunoconjugate.

Chemotherapeutic agents useful in the generation of such immunoconjugates have been described above. Enzymatically active toxins and fragments thereof that can be used include, diphtheria A chain, nonbinding active fragments of diphtheria toxin, exotoxin A chain (from *Pseudomonas aeruginosa),* ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.

Conjugates of the antibody and cytotoxic agent may be made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

Conjugates of an antibody and one or more small molecule toxins, such as a calicheamicin, maytansinoids, a trichothene, and CC1065, and the derivatives of these toxins that have toxin activity, are also contemplated herein.

### Maytansine and maytansinoids

In one embodiment, an immunoconjugate comprises an anti-c-Myb antibody conjugated to one or more maytansinoid molecules. Maytansinoids are mitototic inhibitors which act by inhibiting tubulin polymerization. Maytansine was first isolated from the east African shrub *Maytenus serrata* (U.S. Patent No. 3,896,111). Subsequently, it was discovered that certain microbes also produce maytansinoids, such as maytansinol and C-3 maytansinol esters (U.S. Patent No. 4,151,042). Synthetic maytansinol and derivatives and analogues thereof are disclosed, for example, in U.S. Patent Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533,

In an attempt to improve their therapeutic index, maytansine and maytansinoids have been conjugated to antibodies that bind to antigens on the surface of tumor cells. Immunoconjugates containing maytansinoids and their therapeutic use are disclosed, for example, in U.S. Patent Nos. 5,208,020, 5,416,064 and European Patent EP 0 425 235 B1, . Liu et al., Proc. Natl. Acad. Sci. USA 93:8618-8623 (1996) described immunoconjugates comprising a maytansinoid designated DM1 linked to the monoclonal antibody C242 directed against human gastric cancer. The conjugate was found to be highly cytotoxic towards cultured colon cancer cells, and showed antitumor activity in an *in vivo* tumor growth assay. Chari et al., Cancer Research 52:127-131 (1992) described immunoconjugates in which a maytansinoid was conjugated via a disulfide linker to the murine antibody A7 binding to an antigen on human colon cancer cell lines, or to another murine monoclonal antibody TA.1 that binds the HER-2/*neu* oncogene. The cytotoxicity of the TA. 1-maytansinoid conjugate was tested *in vitro* on the human breast cancer cell line SK-BR-3, which expresses 3x10⁵ HER-2 surface antigens per cell. The drug conjugate achieved a degree of cytotoxicity similar to the free maytansonid drug, which could be increased by increasing the number of maytansinoid molecules per antibody molecule. The A7-maytansinoid conjugate showed low systemic cytotoxicity in mice.

Anti-c-Myb antibody-maytansinoid conjugates are prepared by chemically linking an anti-c-Myb antibody to a maytansinoid molecule without significantly diminishing the biological activity of either the antibody or the maytansinoid molecule. An average of 3-4 maytansinoid molecules conjugated per antibody molecule has shown efficacy in enhancing cytotoxicity of target cells without negatively affecting the function or solubility of the antibody, although even one molecule of toxin per antibody would be expected to enhance cytotoxicity over the use of naked antibody. Maytansinoids are well known in the art and can be synthesized using known techniques or isolated from natural sources. Suitable maytansinoids are disclosed, for example, in U.S. Patent No. 5,208,020 and in the other patents and nonpatent publications referred to hereinabove. Preferred maytansinoids are maytansinol and maytansinol analogues modified in the aromatic ring or at other positions of the maytansinol molecule, such as various maytansinol esters.

There are many linking groups known in the art for making antibody-maytansinoid conjugates, including, for example, those disclosed in U.S. Patent No. 5,208,020 or EP Patent 0 425 235 B1, and Chari et al., Cancer Research 52:127-131 (1992). The tinking groups include disufide groups, thioether groups, acid labile groups, photolabile groups, peptidase labile groups, or esterase labile groups, as disclosed in the above-identified patents, disulfide and thioether groups being preferred.

Conjugates of the antibody and maytansinoid may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Certain coupling agents, including N-succinimidyl-3-(2-pyridyldithio) propionate (SPDP) (Carlsson et al., Biochem. J. 173:723-737 [1978]) and N-succinimidyl-4-(2-pyridylthio)pentanoate (SPP), provide for a disulfide linkage.

The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hyrdoxymethyl, the C-15 position modified with a hydroxyl group, and the C-20 position having a hydroxyl group. In a preferred embodiment, the linkage is formed at the C-3 position of maytansinol or a maytansinol analogue.

### Auristatins and dolastatins

In some embodiments, an immunoconjugate comprises an anti-c-Myb antibody conjugated to a dolastatin or dolostatin peptidic analog or derivative, e.g., an auristatin (US Patent Nos. 5635483; 5780588). Dolastatins and auristatins have been shown to interfere with microtubule dynamics, GTP hydrolysis, and nuclear and cellular division (Woyke et al (2001) Antimicrob. Agents and Chemother. 45(12):3580-3584) and have anticancer (US Pat. No. 5663149) and antifungal activity (Pettit et al (1998) Antimicrob. Agents chemother. 42:2961-2965). The dolastatin or auristatin drug moiety may be attached to the antibody through the N (amino) terminus or the C (carboxyl) terminus of the peptidic drug moiety (WO 02/088172).

Exemplary auristatin embodiments include the N-terminus linked monomethylauristatin drug moieties DE and DF, disclosed in "Monomethylvaline Compounds Capable of Conjugation to Ligands," US Patent Application Publication No. US 2005-0238649 A1,

Typically, peptide-based drug moieties can be prepared by forming a peptide bond between two or more amino acids and/or peptide fragments. Such peptide bonds can be prepared, for example, according to the liquid phase synthesis method (see E. Schröder and K. Lübke, "The Peptides", volume 1, pp 76-136, 1965, Academic Press) that is well known in the field of peptide chemistry. The auristatin/dolastatin drug moieties may be prepared according to the methods of: US 5635483; US 5780588; Pettit et al (1989) J. Am. Chem. Soc. 111:5463-5465; Pettit et al (1998) Anti-Cancer Drug Design 13:243-277; Pettit, G.R, et al. Synthesis, 1996, 719-725; and Pettit et al (1996) J. Chem. Soc. Perkin Trans. 15:859-863. See also Doronina (2003) Nat. Biotechnol. 21(7):778-784; US Patent Application Publication No. 2005-0238649 A1, (disclosing, e.g., linkers and methods of preparing monomethylvaline compounds such as MMAE and MMAF conjugated to linkers).

### Calicheamicin

Another immunoconjugate of interest comprises an anti-c-Myb antibody conjugated to one or more calicheamicin molecules. The calicheamicin family of antibiotics are capable of producing double-stranded DNA breaks at sub-picomolar concentrations. For the preparation of conjugates of the calicheamicin family, see U.S. patents 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, γ₁^{I}, α₂^{I}, α₃^{I}, N-acetyl-γ₁^{I}, PSAG and θ^{I}1 (Hinman et al., Cancer Research 53:3336-3342 (1993), Lode et al., Cancer Research 58:2925-2928 (1998) and the aforementioned U.S. patents to American Cyanamid). Another anti-tumor drug to which the antibody can be conjugated is QFA which is an antifolate. Both calicheamicin and QFA have intracellular sites of action and do not readily cross the plasma membrane. Therefore, cellular uptake of these agents through antibody mediated internalization greatly enhances their cytotoxic effects.

### Other cytotoxic agents

Other antitumor agents that can be conjugated to an anti-c-Myb antibody include BCNU, streptozoicin, vincristine and 5-fluorouracil, the family of agents known collectively as LL-E33288 complex described in U.S. patents 5,053,394, 5,770,710, as well as esperamicins (U.S. patent 5,877,296).

Enzymatically active toxins and fragments thereof which can be used include diphtheria A chain, nonbinding active fragments of diphtheria toxin, exotoxin A chain (from *Pseudomonas aeruginosa),* ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

In another aspect, an immunoconjugate may comprise an anti-c-Myb antibody and a compound with nucleolytic activity (e.g., a ribonuclease or a DNA endonuclease such as a deoxyribonuclease; DNase).

For selective destruction of a tumor, an immunoconjugate may comprise an anti-c-Myb antibody and a highly radioactive atom. A variety of radioactive isotopes are available for the production of radioconjugated anti-c-Myb antibodies. Examples include At²¹¹, I¹³¹, I¹²⁵, Y⁹⁰. Re¹⁸⁶. Re¹⁸⁸_{.} Sm¹⁵³, Bi²¹², P³², Pb²¹² and radioactive isotopes of Lu. When the conjugate is used for diagnosis, it may comprise a radioactive atom for scintigraphic studies, for example tc^{99m} or I¹²³, or a spin label for nuclear magnetic resonance (NMR) imaging (also known as magnetic resonance imaging, mri), such as iodine-123 again, iodine-131, indium-111, fluurine-19. carbon-13, nitrogen-15, oxygen-17, gadolinium, manganese or iron.

The radio- or other labels may be incorporated in the immunoconjugate in known ways. For example, the peptide may be biosynthesized or may be synthesized by chemical amino acid synthesis using suitable amino acid precursors involving, for example, fluorine-19 in place of hydrogen. Labels such as tc^{99m} or I¹²³, Re¹⁸⁶, Re¹⁸⁸ and In¹¹¹ can be attached via a cysteine residue in the peptide. Yttrium-90 can be attached via a lysine residue. The IODOGEN method (Fraker et al (1978) Biochem. Biophys. Res. Commun. 80: 49-57 can be used to incorporate iodine-123. "Monoclonal Antibodies in Immunoscintigraphy" (Chatal.CRC Press 1989) describes other methods in detail.

### 4. Additional Therapeutic Agents

Pharmaceutical formulations may optionally comprise at least one additional therapeutic agent (i.e.. in addition to a c-Myb antagonist, cytotoxic antibody, or immunoconjugate). Such additional therapeutic agents are described in further detail below, Part C.

### 5. Preparation of Pharmaceutical Formulations

Pharmaceutical formulations comprising any of the above agents are prepared for storage by mixing the antibody or immunoconjugate having the desired degree of purity with optional physiologically acceptable carriers, excipients or stabilizers (Remington's Pharmaceutical Sciences 16th edition, Osol, A. Ed. (1980)) in the form of aqueous solutions or lyophilized or other dried formulations. Acceptable carriers, excipients, or stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride); phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*., Zn-protein complexes); and/or non-ionic surfactants such as TWEEN™, PLURONICS™ or polyethylene glycol (PEG). Pharmaceutical formulations to be used for *in vivo* administration are generally sterile. This is readily accomplished by filtration through sterile filtration membranes.

An agent may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the agent of interest, which matrices are in the form of shaped articles, *e.g*., films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated agents remain in the body for a long time, they may denature or aggregate as a result of exposure to moisture at 37°C, resulting in a loss of biological activity and, for antibodies, possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### C. Methods of Treatment and Related Methods

Therapeutic methods using any of the above compositions or pharmaceutical formulations are provided. Such methods include in vitro, ex vivo, and/or in vivo therapeutic methods, unless otherwise indicated.

In one aspect, the invention provides a method of inhibiting the proliferation of a gastric cancer cell, the method comprising exposing the cell to an agent comprising any of the above compositions or pharmaceutical formulations. "Exposing" the cell to an agent encompasses providing the agent extracellularly, intracellularly, or both. In one particular aspect, the invention provides a method of inhibiting the proliferation of a gastric cancer cell, the method comprising exposing the cell to a c-Myb antagonist. In certain embodiments, the c-Myb gene is amplified and/or overexpressed in the gastric cancer cell. In certain embodiments, the gastric cancer cell is derived from a gastric tumor, e.g., a gastric tumor in which the c-Myb gene is amplified and/or overexpressed. In certain embodiments, the gastric cancer cell may be of any of the following cell lines: SNU-1, SNU-5, SNU-16, SNU-484, SNU-601, SNU-620, SNU-638, and SNU-668.

"Inhibiting the proliferation" means decreasing a cell's proliferation by at least 10%, 20%, 30%, 40%, 50%, 60%, 70%, 80%, 90%, 95%, or 100%, and includes inducing cell death. Inhibition of cell proliferation may be measured using methods known to those skilled in the art. For example, a convenient assay for measuring cell proliferation is the CellTiter-Glo^{™} Luminescent Cell Viability Assay, which is commercially available from Promega (Madison, WI). That assay determines the number of viable cells in culture based on quantitation of ATP present, which is an indication of metabolically active cells. *See* Crouch et al (1993) J. Immunol. Meth. 160:81-88, US Pat. No. 6602677. The assays may be conducted in 96- or 384-well format, making it amenable to automated high-throughput screening (HTS). *See* Cree et al (1995) AntiCancer Drugs 6:398-404. The assay procedure involves adding a single reagent (CellTiter-Glo^{®} Reagent) directly to cultured cells. This results in cell lysis and generation of a luminescent signal produced by a luciferase reaction. The luminescent signal is proportional to the amount of ATP present, which is directly proportional to the number of viable cells present in culture. Data can be recorded by luminometer or CCD camera imaging device. The luminescence output is expressed as relative light units (RLU).

In another aspect, a method of treating a gastric cancer is provided, the method comprising administering to an individual having the gastric cancer an effective amount of any of the above compositions or pharmaceutical formulations. In certain embodiments, the pharmaceutical formulation comprises a c-Myb antagonist. In certain embodiments, the gastric cancer is associated with amplification and/or overexpression of the c-Myb gene. In certain embodiments, the individual is a non-human animal model for gastric cancer. Mouse models of gastric cancer are discussed, for example, in McCarty et al. (2004) British Journal of Cancer 90:705-711. In certain embodiments, the individual is a human. In certain embodiments, an effective amount of the pharmaceutical formulation results in any one of the following: reduction in the number of cancer cells or elimination of the cancer cells; reduction in the tumor size; full or partial inhibition of cancer cell infiltration into peripheral organs, including the spread of cancer into soft tissue and bone; full or partial inhibition of tumor metastasis; full or partial inhibition of tumor growth; and/or full or partial relief of one or more of the symptoms associated with the cancer; and reduced morbidity and mortality.

In certain embodiments, a composition or pharmaceutical formulation as provided above is administered in combination with at least one additional therapeutic agent and/or adjuvant. In certain embodiments, an additional therapeutic agent is a cytotoxic agent, a chemotherapeutic agent, or a growth inhibitory agent. In one of such embodiments, a chemotherapeutic agent is an agent or a combination of agents used in the treatment of gastric cancer. Such agents include, but are not limited to, fluorouracil (5FU), folinic acid, heptaplatin, cisplatin, taxanes, camptothecins, fluoropyrimidines, or combinations thereof.

Such combination therapies noted above encompass combined administration (where two or more therapeutic agents are included in the same or separate formulations), and separate administration, in which case, administration of a c-Myb antagonist can occur prior to, simultaneously, and/or following, administration of the additional therapeutic agent and/or adjuvant. A c-Myb antagonist can also be used in combination with radiation therapy.

An agent comprising a composition or pharmaceutical formulation described herein (and any additional therapeutic agent or adjuvant) can be administered by any suitable means, including parenteral, subcutaneous, intraperitoneal, intrapulmonary, and intranasal, and, if desired for local treatment, intralesional administration. Parenteral infusions include intramuscular, intravenous, intraarterial, intraperitoneal, or subcutaneous administration. In addition, an agent is suitably administered by pulse infusion, particularly with declining doses of the agent. Dosing can be by any suitable route, e.g. by injections, such as intravenous or subcutaneous injections, depending in part on whether the administration is brief or chronic.

Gene therapy methods may be used, e.g., to deliver any of the nucleic acids described herein to cells in vivo. In certain embodiments of gene therapy, the nucleic acid encodes a polypeptide antagonist of c-Myb, e.g., an antibody that binds to c-Myb. According to one embodiment, a targeting agent is used to direct the vehicle containing the nucleic acid to a desired tissue.

Presently, there are generally two major approaches to getting a nucleic acid (optionally contained in a vector) into a mammal's cells: *in vivo* and *ex vivo.* For *in vivo* delivery the nucleic acid is injected directly into a mammal, usually at the sites where the nucleic acid and, if applicable, the encoded polypeptide, are desired. For *ex vivo* treatment, the mammal's cells are removed, the nucleic acid is introduced into these isolated cells, and the modified cells are administered to the mammal either directly or, for example, encapsulated within porous membranes that are implanted into the mammal (*see, e.g.,* U.S. Pat. Nos. 4,892,538 and 5,283,187).

There are a variety of techniques available for introducing nucleic acids into viable cells. The techniques vary depending upon whether the nucleic acid is transferred into cultured cells *in vitro,* or transferred *in vivo* in the cells of the intended host. Techniques suitable for the transfer of nucleic acid into mammalian cells *in vitro* include the use of liposomes, electroporation, microinjection, transduction, cell fusion, DEAE-dextran, the calcium phosphate precipitation method, etc. Transduction involves the association of a replication-defective, recombinant viral (including, but not limited to, retroviral) particle with a cellular receptor, followed by introduction of the nucleic acids contained in the particle into the cell. A commonly used vector for *ex vivo* delivery of a nucleic acid is a retrovirus.

Commonly used *in vivo* nucleic acid transfer techniques include transfection with viral or non-viral vectors (such as adenovirus, lentivirus, Herpes simplex I virus, or adeno-associated virus (AAV)) and lipid-based systems (useful lipids for lipid-mediated transfer of a nucleic acid are, for example, DOTMA, DOPE, and DC-Chol; *see, e.g.,* Tonkinson et al., Cancer Investigation, 14(1): 54-65 (1996)). Such vectors are used to synthesize virus that can be used as vehicles for delivering agents, such as antagonists and nucleic acid molecules of this invention. The most commonly used vectors for use in gene therapy are viruses, e.g., adenoviruses, AAV, lentiviruses, or retroviruses. In one embodiment, a viral vector such as a retroviral vector includes at least one transcriptional promoter/enhancer or locus-defining element(s), or other elements that control gene expression by other means such as alternate splicing, nuclear RNA export, or post-translational modification of messenger. In addition, a viral vector such as a retroviral vector may include a nucleic acid molecule that is operably linked to a nucleic acid of interest and acts as a translation initiation sequence. Such vector constructs may also include a packaging signal, long terminal repeats (LTRs) or portions thereof, and positive and negative strand primer binding sites appropriate to the virus used (if these are not already present in the viral vector). In addition, such vector constructs may include a signal sequence for secretion of the encoded polypeptide from a host cell in which it is placed. Optionally, the vector construct can also include a signal that directs polyadenylation, as well as one or more restriction sites and a translation termination sequence. By way of example, vectors will typically include a 5' LTR, a tRNA binding site, a packaging signal, an origin of second-strand DNA synthesis, and a 3' LTR or a portion thereof. Other vectors that can be used are non-viral, such as cationic lipids, polylysine, and dendrimers.

In some situations, the vehicle used for delivery of a nucleic acid or other molecule is associated with a targeting agent that targets the vehicle to specific cell populations. In one embodiment, the targeting agent is an antibody specific for a cell-surface membrane protein on the target cell, a ligand for a receptor on the target cell, etc. Where liposomes are employed, proteins that bind to a cell-surface membrane protein associated with endocytosis can be used for targeting and/or to facilitate uptake. The technique of receptor-mediated endocytosis is described, for example, by Wu et a/., J. Biol. Chem., 262: 4429-4432 (1987); and Wagner et al., Proc. Natl. Acad. Sci. USA, 87: 3410-3414 (1990).

For a review of the currently known gene marking and gene therapy protocols, *see,* Anderson et al, Science, 256: 808-813 (1992). *See* also WO 93/25673 and the references cited therein. Suitable gene therapy and methods for making retroviral particles and structural proteins can be found in, *e.g*., U.S. Pat. No. 5,681,746.

In certain embodiments in which the c-Myb antagonist is an antisense nucleic acid (e.g., an ODN), guidance for dosage and in vivo administration of antisense nucleic acids may be found in Khan et al. (2004) J. Drug Targeting 12:393-404*.*

### III. EXAMPLES

### A. Samples

Nine fresh frozen gastric tumors, each from a different patient sample, were selected for analysis. Five of the nine were gastrointestinal stromal tumors (GISTS), and four of the nine were gastric adenocarcinomas. Each tumor sample had greater than 75% neoplastic cell content, as estimated by a pathologist. From each tumor both RNA and DNA were extracted and purified by standard methods.

### B. DNA copy number analysis

The GeneChip® Human Mapping 500K Array Set (Affymetrix, Santa Clara, CA) was used to measure DNA copy number changes in the gastric tumors. The Gene Chip® Human Mapping 500K Array Set consists of two arrays (the 250K "Sty I" array and the 250K "Nsp I" array), each containing probes specific for approximately 250,000 SNPs, for a total of approximately 500,000 SNPs. The SNPs are distributed throughout the genome, thereby permitting a genome-wide analysis of DNA copy number. Each array in the array set includes more than 6.5 million features, with each feature consisting of over I million copies of a 25-bp oligonucleotide of defined sequence.

From each tumor sample, DNA was amplified, labeled, and digested with either Sty I or Nsp I as per Affymetrix's standard protocols, and the resulting preparation was allowed to hybridize to both arrays of the GeneChip® Human Mapping 500K Array Set.

Hybridization to the microarrays was detected according to Affymetrix's standard protocols, and intensity values for each feature were generated. Intensity values were normalized to a reference set of normal genomic DNA. Features were then mapped to the human genome. Thus, the normalized intensity values reflected the DNA copy number at a particular genomic locus.

Figure 1 shows the results of copy number analysis at a region of chromosome 6. Tumor samples arc listed by tumor type (GIST or adenocarcinoma ("Adeno")) and numerical designation (e.g.. "X31131") at the left of the graph. The graph shows the normalized intensity values from the DNA copy number analysis for each tumor, with each feature being represented as a vertical line. For each tumor, the vertical lines are plotted along a horizontal axis, which represents a region of chromosome 6 from about 130,000,000 to 140,000,000 nucleotides. The height of each vertical line reflects the normalized intensity value, which is a measure of the DNA copy number at that point on the chromosome. A spike of signal intensity was observed between 135,000,000 and 136,000,000 nucleotides for two of the four adenocarcinomas. X10253 and X 10227. The normalized intensity value for X 10253 and X 10227 was increased by at least about 5-10 fold at that region.

### C. Expression analysis

The GeneChip® Human Genome U133A 2.0 Array and the GeneChip® Human Genome U133 Plus 2.0 Array (Affymetrix, Santa Clara, CA) were used to measure mRNA expression in X10253 and X 10227. Purified RNA samples were reverse transcribed, amplified. labeled. and otherwise treated as per Affymetrix's standard protocols and allowed to hybridize to one or the other of the arrays. Hybridization to the arrays was detected according to Affymetrix's standard methods, and intensity values for each feature were generated. The intensity value for each feature was normalized to the median intensity of that feature across all tumor samples. Features were then mapped to the corresponding coding regions in the genome. Thus, the normalized intensity values reflected mRNA expression levels for each feature, and each feature was correlated with a particular position in the genome.

Figure 2 shows the results of the copy number analysis (taken from Figure 1) and mRNA expression analysis for X10253 and X10227. For the expression analysis, normalized intensity values corresponding to mRNA expression levels are shown as vertical lines along the horizontal axes representing the indicated region of chromosome 6. The height of each vertical line reflects the relative mRNA expression level for each feature. The coding regions of genes known to map to the indicated region of chromosome 6 are shown below the copy number and expression axes. Thus, Figure 2 shows the copy number and relative mRNA expression levels corresponding to coding regions that occur within the indicated region of chromosome 6.

The c-Myb gene falls squarely within the region of increased copy number between 135,000,000 and 136,000,000 nucleotides in X10253 and X10227. The increase in DNA copy number of the c-Myb gene is correlated with marked overexpression (at least about 5-10 fold overexpression) of the c-Myb transcript.

The high level amplification of the c-Myb gene suggests that an increase in copy number of that gene causes overexpression of the encoded proto-oncogene, thereby promoting the growth and proliferation of gastric tumor cells. The observed overexpression of c-Myb mRNA is consistent with that conclusion.

Although the foregoing invention has been described in some detail by way of illustration and example for purposes of clarity of understanding, the descriptions and examples should not be construed as limiting the scope of the invention.

## Claims

1. A method of diagnosing the presence of a gastric cancer in a mammal, the method comprising detecting whether the c-Myb gene is amplified in a test gastric sample from the mammal relative to a control sample, wherein amplification of the c-Myb gene indicates the presence of gastric cancer in the mammal.

2. The method of claim 1, wherein detecting whether the c-Myb gene is amplified comprises detecting whether the copy number of the c-Myb gene is increased by at least 5-fold.

3. A method of diagnosing the presence of a gastric cancer in a mammal, the method comprising detecting expression of the c-Myb gene in a test gastric sample from the mammal, wherein a higher level of c-Myb gene expression in the test gastric sample relative to a control sample indicates the presence of gastric cancer in the mammal.

4. The method of claim 3, wherein detecting expression of the c-Myb gene comprises determining the level of mRNA transcription from the c-Myb gene.

5. The method of claim 4, wherein a higher level of c-Myb gene expression comprises at least a 5-fold increase in mRNA transcription from the c-Myb gene in the test gastric sample relative to the control sample.

6. The method of claim 3, wherein detecting expression of the c-Myb gene comprises determining the level of c-Myb.

7. The method of claim 6, wherein detecting expression of the c-Myb gene comprises contacting the test gastric sample with an anti-c-Myb antibody and determining the level of expression of c-Myb in the test gastric sample by detecting binding of the anti-c-Myb antibody to c-Myb.

8. The method of claim 6, wherein a higher level of c-Myb gene expression comprises at least a 5-fold increase in c-Myb levels.

9. A c-Myb antagonist for use in a method of treating a gastric cancer associated with amplification or overexpression of the c-Myb gene, the method comprising administering to an individual having the gastric cancer an effective amount of a pharmaceutical formulation comprising the c-Myb antagonist, wherein the c-Myb antagonist is selected from:
an antisense nucleic acid that binds to the nucleic acid encoding c-Myb; a nucleic acid that binds to the DNA binding domain of c-Myb; a nucleic acid encoding an antibody or an antibody fragment that binds to c-Myb; and an antibody or antibody fragment that binds to c-Myb.

10. The c-Myb antagonist for use according to claim 9, wherein the c-Myb antagonist is an antisense nucleic acid of 10-30 nucleotides in length that binds to and reduces expression of a nucleic acid encoding c-Myb.

11. The c-Myb antagonist for use according to claim 10, wherein the antisense nucleic acid is an oligodeoxynucleotide.

12. The c-Myb antagonist for use according to claim 9, wherein the c-Myb antagonist is a nucleic acid that binds to the DNA binding domain of c-Myb.

13. The c-Myb antagonist for use according to claim 9, wherein the c-Myb antagonist is a nucleic acid encoding an antibody or antibody fragment.

14. The c-Myb antagonist for use according to claim 13, wherein the antibody fragment that binds to c-Myb is an scFv.

15. A method for determining whether an individual having a gastric cancer will respond to a therapeutic that targets c-Myb or the c-Myb gene, the method comprising determining whether the c-Myb gene is amplified in the gastric cancer, wherein amplification of the c-Myb gene indicates that the individual will respond to the therapeutic.

16. The method of claim 15, wherein the therapeutic is selected from an antisense nucleic acid; a nucleic acid that binds to the DNA binding domain of c-Myb; a small organic molecule that binds to c-Myb; and a nucleic acid encoding an antibody that binds to c-Myb.

## Patentansprüche

1. Verfahren zur Diagnose des Vorhandenseins eines Magenkrebses bei einem Säugetier, wobei das Verfahren das Detektieren, ob in einer Magentestprobe aus dem Säugetier im Vergleich zu einer Kontrollprobe das c-Myb-Gen amplifiziert wird, umfasst, worin die Amplifikation des c-Myb-Gens ein Indikator für das Vorhandensein von Magenkrebs bei dem Säugetier ist.

2. Verfahren nach Anspruch 1, worin das Detektieren, ob das c-Myb-Gen amplifiziert wird, das Detektieren, ob die Kopienanzahl des c-Myb-Gens um das zumindest 5fache erhöht ist, umfasst.

3. Verfahren zur Diagnose des Vorhandenseins eines Magenkrebses bei einem Säugetier, wobei das Verfahren die Detektion der Expression des c-Myb-Gens in einer Magentestprobe aus dem Säugetier umfasst, worin ein höheres Ausmaß an Expression des c-Myb-Gens in der Magentestprobe im Vergleich zu einer Kontrollprobe ein Indikator für das Vorhandensein von Magenkrebs bei dem Säugetier ist.

4. Verfahren nach Anspruch 3, worin das Detektieren der Expression des c-Myb-Gens das Bestimmen des Ausmaßes an mRNA-Transkription von dem c-Myb-Gen umfasst.

5. Verfahren nach Anspruch 4, worin ein höheres Ausmaß an Expression des c-Myb-Gens eine zumindest 5-mal höhere mRNA-Transkription von dem c-Myb-Gen in der Magentestprobe im Vergleich zur Kontrollprobe umfasst.

6. Verfahren nach Anspruch 3, worin das Detektieren der Expression des c-Myb-Gens das Bestimmen der Menge an c-Myb umfasst.

7. Verfahren nach Anspruch 6, worin das Detektieren der Expression des c-Myb-Gens das Kontaktieren der Magentestprobe mit einem Anti-c-Myb-Antikörper und das Bestimmen des Ausmaßes an Expression von c-Myb in der Magentestprobe durch Detektieren der Bindung des Anti-c-Myb-Antikörpers an c-Myb umfasst.

8. Verfahren nach Anspruch 6, worin ein höheres Ausmaß an Expression des c-Myb-Gens eine zumindest 5fache Erhöhung der c-Myb-Mengen umfasst.

9. c-Myb-Antagonist zur Verwendung in einem Verfahren zur Behandlung eines Magenkrebses, der mit Amplifikation oder Überexpression des c-Myb-Gens assoziiert ist, wobei das Verfahren das Verabreichen einer wirksamen Menge einer pharmazeutischen Formulierung, die den c-Myb-Antagonisten umfasst, an ein Individuum mit Magenkrebs umfasst, worin der c-Myb-Antagonist aus den folgenden ausgewählt ist:
einer Antisense-Nucleinsäure, die an die für c-Myb kodierende Nucleinsäure bindet; einer Nucleinsäure, die an die DNA-Bindedomäne von c-Myb bindet; einer Nucleinsäure, die für einen Antikörper oder ein Antikörperfragment, der bzw. das an c-Myb bindet, kodiert; und einem Antikörper oder Antikörperfragment, der bzw. das an c-Myb bindet.

10. c-Myb-Antagonist zur Verwendung nach Anspruch 9, worin der c-Myb-Antagonist eine Antisense-Nucleinsäure mit einer Länge von 10 bis 30 Nucleotiden ist, die an eine für c-Myb kodierende Nucleinsäure bindet und ihre Expression reduziert.

11. c-Myb-Antagonist zur Verwendung nach Anspruch 10, worin die Antisense-Nucleinsäure ein Oligodesoxynucleotid ist.

12. c-Myb-Antagonist zur Verwendung nach Anspruch 9, worin der c-Myb-Antagonist eine Nucleinsäure ist, die an die DNA-Bindedomäne von c-Myb bindet.

13. c-Myb-Antagonist zur Verwendung nach Anspruch 9, worin der c-Myb-Antagonist eine Nucleinsäure ist, die für einen Antikörper oder ein Antikörperfragment kodiert.

14. c-Myb-Antagonist zur Verwendung nach Anspruch 13, worin das Antikörperfragment, das an c-Myb bindet, ein scFv ist.

15. Verfahren zur Bestimmung, ob ein Individuum mit Magenkrebs auf ein Therapeutikum ansprechen wird, das auf c-Myb oder das c-Myb-Gen gerichtet ist, wobei das Verfahren das Bestimmen, ob das c-Myb-Gen im Magenkrebs amplifiziert wird, umfasst, worin die Amplifikation des c-Myb-Gens ein Indikator dafür ist, dass das Individuum auf das Therapeutikum ansprechen wird.

16. Verfahren nach Anspruch 15, worin das Therapeutikum aus einer Antisense-Nucleinsäure, einer Nucleinsäure, die an die DNA-Bindedomäne von c-Myb bindet, einem kleinen organischen Molekül, das an c-Myb bindet, und einer Nucleinsäure, die für einen Antikörper kodiert, der an c-Myb bindet, ausgewählt ist.

## Revendications

1. Procédé de diagnostic de la présence d'un cancer gastrique chez un mammifère, le procédé comprenant la détection si le gène c-Myb est amplifié dans un échantillon gastrique de test du mammifère relativement à un échantillon de contrôle, où l'amplification du gène c-Myb indique la présence du cancer gastrique chez le mammifère.

2. Procédé selon la revendication 1, dans lequel la détection si la gène c-Myb est amplifié comprend la détection si le nombre de copies du gène c-Myb est augmenté selon au moins 5 fois.

3. Procédé de diagnostic de la présence d'un cancer gastrique chez un mammifère, le procédé comprenant la détection de l'expression du gène c-Myb dans un échantillon gastrique de test du mammifère, où un niveau plus élevé de l'expression du gène c-Myb dans l'échantillon gastrique de test relativement à un échantillon de contrôle indique la présence d'un cancer gastrique chez le mammifère.

4. Procédé selon la revendication 3, dans lequel la détection de l'expression du gène c-Myb comprend la détermination du niveau de la transcription de ARNm du gène c-Myb.

5. Procédé selon la revendication 4, dans lequel un niveau plus élevé de l'expression du gène c-Myb comprend une augmentation d'au moins 5 fois dans la transcription de ARNm du gène c-Myb dans l'échantillon gastrique de test relativement à l'échantillon de contrôle.

6. Procédé selon la revendication 3, dans lequel la détection de l'expression du gène c-Myb comprend la détermination du niveau de c-Myb.

7. Procédé selon la revendication 6, dans lequel la détection de l'expression du gène c-Myb comprend la mise en contact de l'échantillon gastrique de test avec un anticorps anti-c-Myb et la détermination du niveau de l'expression de c-Myb dans l'échantillon gastrique de test en détectant la liaison de l'anticorps anti-c-Myb à c-Myb.

8. Procédé selon la revendication 6, dans lequel un niveau plus élevé de l'expression du gène c-Myb comprend une augmentation d'au moins 5 fois dans les niveaux de c-Myb.

9. Antagoniste du c-Myb pour utilisation dans une méthode de traitement d'un cancer gastrique associé à l'amplification ou à la surexpression du gène c-Myb, la méthode comprenant l'administration à un individu, ayant le cancer gastrique, d'une quantité efficace d'une formulation pharmaceutique comprenant l'antagoniste de c-Myb, où l'antagoniste de c-Myb est sélectionné parmi:
un acide nucléique antisens qui se lie à l'acide nucléique codant c-Myb; un acide nucléique qui se lie au domaine de liaison d'ADN du c-Myb; un acide nucléique codant pour un anticorps ou un fragment d'anticorps qui se lie au c-Myb; et un anticorps ou un fragment d'anticorps qui se lie au c-Myb.

10. Antagoniste du c-Myb pour utilisation selon la revendication 9, où l'antagoniste du c-Myb est un acide nucléique antisens de 10-30 nucléotides en longueur qui se lie à et réduit l'expression d'un acide nucléique codant c-Myb.

11. Antagoniste du c-Myb pour utilisation selon la revendication 10, où l'acide nucléique antisens est un oligodésoxynucléotide.

12. Antagoniste du c-Myb pour utilisation selon la revendication 9, où l'antagoniste du c-Myb est un acide nucléique qui se lie au domaine de liaison d'ADN du c-Myb.

13. Antagoniste du c-Myb pour utilisation selon la revendication 9, où l'antagoniste du c-Myb est un acide nucléique codant pour un anticorps ou un fragment d'anticorps.

14. Antagoniste du c-Myb pour utilisation selon la revendication 13, où le fragment d'anticorps qui se lie au c-Myb est un scFv.

15. Méthode pour déterminer si un individu ayant un cancer gastrique répondra à une thérapeutique qui cible c-Myb ou le gène du c-Myb, la méthode comprenant la détermination si le gène de c-Myb est amplifié dans le cancer gastrique, où l'amplification du gène c-Myb indique que l'individu répondra à la thérapeutique.

16. Méthode selon la revendication 15, dans laquelle la thérapeutique est sélectionnée parmi un acide nucléique antisens; un acide nucléique qui se lie au domaine de liaison d'ADN du c-Myb; une petite molécule organique qui se lie au c-Myb; et un acide nucléique codant pour un anticorps qui se lie au c-Myb.
